(19) Europäisches Patentamt
European Patent Office
Office européen des brevets

(11) **EP 4 317 956 A1**

(12) **EUROPEAN PATENT APPLICATION**
published in accordance with Art. 153(4) EPC

(43) Date of publication:
**07.02.2024 Bulletin 2024/06**

(21) Application number: **22787873.3**

(22) Date of filing: **28.02.2022**

(51) International Patent Classification (IPC):
*G01N 27/12* (2006.01)      *A01G 7/00* (2006.01)
*A01M 1/00* (2006.01)

(52) Cooperative Patent Classification (CPC):
**A01G 7/00; A01M 1/00; G01N 27/12**

(86) International application number:
**PCT/JP2022/008215**

(87) International publication number:
**WO 2022/219945 (20.10.2022 Gazette 2022/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.04.2021 JP 2021067823**

(71) Applicant: **Sony Group Corporation
Tokyo 108-0075 (JP)**

(72) Inventors:
• **SUGIYAMA, Taiki
Tokyo 108-0075 (JP)**
• **NAKAO, Michiko
Tokyo 108-0075 (JP)**
• **YAMANO, Ryota
Tokyo 108-0075 (JP)**
• **ISHIDA, Yuichi
Tokyo 108-0075 (JP)**

(74) Representative: **D Young & Co LLP
120 Holborn
London EC1N 2DY (GB)**

(54) **GAS DETECTION METHOD, INFORMATION PROCESSING DEVICE, PLANT DIAGNOSIS METHOD, AND PLANT DIAGNOSIS DEVICE**

(57)      [Object] To provide a gas detection method, an information processing apparatus, a method of diagnosing a plant, and a plant diagnosing apparatus that are capable of easily identifying a specific gas species.

[Solving Means] A gas detection method according to an embodiment of the present technology includes: heating a semiconductor sensor that includes an adsorption layer formed of a metal oxide; measuring a resistance value of the semiconductor sensor in an air atmosphere in which a reducing gas is present; and determining, where the measured resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

FIG.14

**Description**

Technical Field

The present technology relates to a gas detection method, an information processing apparatus, a method of diagnosing a plant, and a plant diagnosing apparatus.

Background Art

A technology for using a metal oxide (MOx) semiconductor sensor to detect and identify a gas has been known. For example, Patent Literature 1 discloses a gas sensor that includes an adsorption layer and a sensing layer, the adsorption layer adsorbing a gas containing a detection target gas and a non-detection target gas, the sensing layer being covered with the adsorption layer, electrical properties of the sensing layer changing depending on the concentration of the detection target gas that has passed through the adsorption layer. Further, Patent Literature 2 disclose a gas detection apparatus that identifies the type of gas on the basis of the output values of a first gas sensor and a second gas sensor.

Citation List

Patent Literature

Patent Literature 1: Japanese Patent Application Laid-open No. 2017-223557
Patent Literature 2: Japanese Patent Application Laid-open No. 2001-175969

Disclosure of Invention

Technical Problem

When a reducing gas is sprayed while heating a MOx semiconductor sensor, the resistance value of the semiconductor sensor generally starts to change from a certain temperature. In this regard, a gas can be detected or quantified from the amount of change (sensitivity) in the resistance value. Further, when there is a large difference in profiles of the amount of change (sensitivity) in the temperature and resistance, the type of gas can be identified. However, in the case where there is no sufficient difference in the profiles, it has been difficult to identify the type of gas.

In view of the circumstances as described above, it is an object of the present technology to provide a gas detection method, an information processing apparatus, a method of diagnosing a plant, and a plant diagnosing apparatus that are capable of easily identifying a specific gas species.

Solution to Problem

A gas detection method according to an embodiment of the present technology includes: heating a semiconductor sensor that includes an adsorption layer formed of a metal oxide.

A resistance value of the semiconductor sensor in an air atmosphere in which a reducing gas is present is measured.

It is determined, where the measured resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

The air atmosphere in which the reducing gas is not present may be dry air.

The semiconductor sensor may include a plurality of semiconductor sensors. In this case, the step of heating the semiconductor sensor includes heating each of the plurality of semiconductor sensors to different heating temperatures, and the step of measuring a resistance value of the semiconductor sensor includes measuring a resistance value of each of the plurality of semiconductor sensors.

The hydrocarbon substance may be a substance containing a C6 linear aliphatic compound.

The hydrocarbon substance may have a molecular weight of 90 or more and 160 or less.

The metal oxide may be a sintered body containing a metal oxide material and a catalyst metal material.

The metal oxide material may include a tungsten oxide, an indium oxide, a tin oxide, or a zinc oxide, and the catalyst metal material may include iridium, an oxide thereof, palladium, an oxide thereof, rhenium, or an oxide thereof.

A gas detection method according to another embodiment of the present technology includes: heating a semiconductor sensor that includes an adsorption layer formed of a metal oxide.

A resistance value of the semiconductor sensor in an air atmosphere in which a reducing gas is present is measured.

It is determined, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from a plant or an animal.

An information processing apparatus according to an embodiment of the present technology includes: an acquisition unit; and a determination unit.

The acquisition unit acquires a resistance value of a metal oxide semiconductor sensor in an air atmosphere in which a reducing gas is present.

The determination unit determines, where the acquired resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

An information processing apparatus according to another embodiment of the present technology includes: an acquisition unit; and a determination unit.

The acquisition unit acquires a resistance value of a metal oxide semiconductor sensor in an air atmosphere in which a reducing gas is present.

The determination unit determines, where sensitivity that is a ratio of a resistance value of the semiconductor sensor in the air atmosphere in which the reducing gas is not present to the acquired resistance value is less than one in a first temperature range and greater than one in a second temperature range higher than the first temperature range, that the reducing gas contains a hydrocarbon substance emitted from a plant or an animal.

A method of diagnosing a plant according to an embodiment of the present technology includes: heating a semiconductor sensor that includes an adsorption layer formed of a metal oxide in an area in which one or more plants to be tested are grown.

A resistance value of the semiconductor sensor in an air atmosphere in which a reducing gas is present is measured.

It is determined, where the measured resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal, and

a state of the plants in the area is diagnosed on the basis of detection information of a hydrocarbon substance emitted from the plants.

A gas detection method according to another embodiment of the present technology includes: heating a semiconductor sensor that includes an adsorption layer formed of a metal oxide in an area in which one or more plants to be tested are grown.

A resistance value of the semiconductor sensor in an air atmosphere in which a reducing gas is present is measured.

It is determined, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from a plant or an animal, and

a state of the plants in the area is diagnosed on the basis of detection information of a hydrocarbon substance emitted from the plants.

A plant diagnosing apparatus according to an embodiment of the present technology includes: an acquisition unit; a determination unit; and a diagnosis unit.

In an area in which one or more plants to be tested are grown,

the acquisition unit acquires a resistance value of a metal oxide semiconductor sensor in an air atmosphere in which a reducing gas is present.

The determination unit determines, where the acquired resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

The diagnosis unit diagnoses a state of the plants in the area on the basis of detection information of a hydrocarbon substance emitted from the plants obtained by the determination unit.

A plant diagnosing apparatus according to another embodiment of the present technology includes: an acquisition unit; a determination unit; and a diagnosis unit.

In an area in which one or more plants to be tested are grown,
the acquisition unit acquires a resistance value of a metal oxide semiconductor sensor in an air atmosphere in which a reducing gas is present.

The determination unit determines, where sensitivity that is a ratio of a resistance value of the semiconductor sensor in the air atmosphere in which the reducing gas is not present to the acquired resistance value is less than one in a first temperature range and greater than one in a second temperature range higher than the first temperature range, that the reducing gas contains a hydrocarbon substance emitted from a plant or an animal.

The diagnosis unit diagnoses a state of the plants in the area on the basis of detection information of a hydrocarbon substance emitted from the plants obtained by the determination unit.

Brief Description of Drawings

[Fig. 1] Fig. 1 is a schematic cross-sectional view showing a configuration of a main part of a semiconductor sensor to be used in an embodiment of the present technology.

[Fig. 2] Fig. 2 is a drive circuit of the semiconductor sensor.

[Fig. 3] Fig. 3 is a diagram showing an example of a time change of a resistance value output from the semiconductor sensor.

[Fig. 4] Fig. 4 is a schematic diagram showing a relationship between a heating temperature of the semiconductor sensor.

[Fig. 5] Fig. 5 is a schematic diagram showing a relationship between a heating temperature and sensitivity of the semiconductor sensor.

[Fig. 6] Fig. 6 is a schematic diagram showing a relationship between a heating temperature of the semiconductor sensor in the presence of a specific gas species.

[Fig. 7] Fig. 7 is a schematic diagram showing a relationship between a heating temperature and sensitivity of the semiconductor sensor in the presence of a specific gas species.

[Fig. 8] Fig. 8 is a schematic diagram describing a method of specifying a gas species using a difference in sensitivity.

[Fig. 9] Fig. 9 is a schematic configuration diagram of a gas detection system.

[Fig. 10] Fig. 10 is an experimental result showing a resistance value and sensitivity of the semiconductor sensor when (A) toluene, (B) benzene, and (C) cyclohexane are used as reducing gases.

[Fig. 11] Fig. 11 is an experimental result showing a resistance value and sensitivity of the semiconductor sensor when (A) n-hexanal, (B) (E)-2-hexenal, (C) (Z)-3-hexenol are used as reducing gases.

[Fig. 12] Fig. 12 is an experimental result showing a resistance value and sensitivity of the semiconductor sensor when (A) (Z)-3-hexenyl acetate and (B) $\alpha$-pinene are used as reducing gases.

[Fig. 13] Fig. 13 is a schematic configuration diagram of gas detection apparatus.

[Fig. 14] Fig. 14 is a flowchart showing an example of the processing procedure of the gas detection apparatus.

[Fig. 15] Fig. 15 is a flowchart showing another example of the processing procedure of the gas detection apparatus.

[Fig. 16] Fig. 16 is a schematic configuration diagram of a plant diagnosing apparatus.

[Fig. 17] Fig. 17 is a flowchart showing an example of the processing procedure of the plant diagnosing apparatus.

[Fig. 18] Fig. 18 is a flowchart showing another example of the processing procedure of the plant diagnosing apparatus.

[Fig. 19] Fig. 19 is a process of determining the presence or absence of plant damage by pests.

[Fig. 20] Fig. 20 is an experimental result showing a resistance value and sensitivity of the semiconductor sensor when methyl salicylate is used as a reducing gas.

Mode(s) for Carrying Out the Invention

An embodiment according to the present technology will be described below with reference to the drawings.

[Semiconductor sensor]

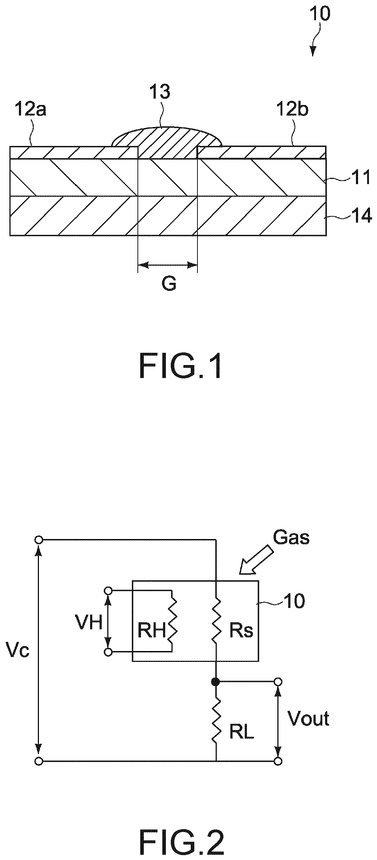

Fig. 1 is a schematic cross-sectional view showing a configuration of a main part of a semiconductor sensor 10 to be used in an embodiment of the present technology.

The semiconductor sensor 10 includes a substrate 11, a pair of electrodes 12a and 12b formed on the front surface of the substrate 11, an adsorption layer 13 provided between the pair of electrodes 12a and 12b, and a heating layer 14 disposed on the back surface of the substrate 11.

The substrate 11 is, for example, an alumina substrate, a silicon substrate, or a quartz substrate.

The pair of electrodes 12a and 12b include, for example, a metal layer of Ti, Au, or s stacked film thereof, and are formed on the front surface of the substrate 11 with a gap G therebetween.

The adsorption layer 13 contains a metal oxide (MOx) including a sintered body containing a metal oxide material and a catalyst metal material. Examples of the metal oxide material include a tungsten oxide, an indium oxide, a tin oxide, and a zinc oxide. Examples of the catalyst metal material include iridium, an oxide thereof, palladium, an oxide thereof, rhenium, and an oxide thereof.

The heating layer 14 is for heating the adsorption layer 13 to a predetermined temperature and include, for example, a ceramic heater. The heating layer 14 is connected to a heater power source (not shown) and is configured to be heated to a temperature of, for example, 500°C or less by a control device described below.

In general, when spraying a reducing gas while heating a MOx semiconductor sensor, the resistance value of the semiconductor sensor generally starts to change from a certain temperature. In this regard, a gas can be detected or quantified from the amount of change (sensitivity) in the resistance value. Further, when there is a large difference in profiles of the amount of change (sensitivity) in the temperature and resistance, the type of gas can be identified.

Fig. 2 is a drive circuit of the semiconductor sensor 10. In the figure, Rs is a sensor resistor, RL is a load resistor connected in series with the sensor resistor Rs, RH is a heater resistor for heating the semiconductor sensor 10, and the heating temperature of the heating layer 14 is adjusted by a heater voltage VH. Then, the sensor resistor Rs can be measured on the basis of a voltage Vout across the load resistor RL when a power supply voltage Vc is applied to both ends of the series resistors (Rs and RL) by the following formula.

```
Rs = ((Vc - Vout) / Vout) × RL
```

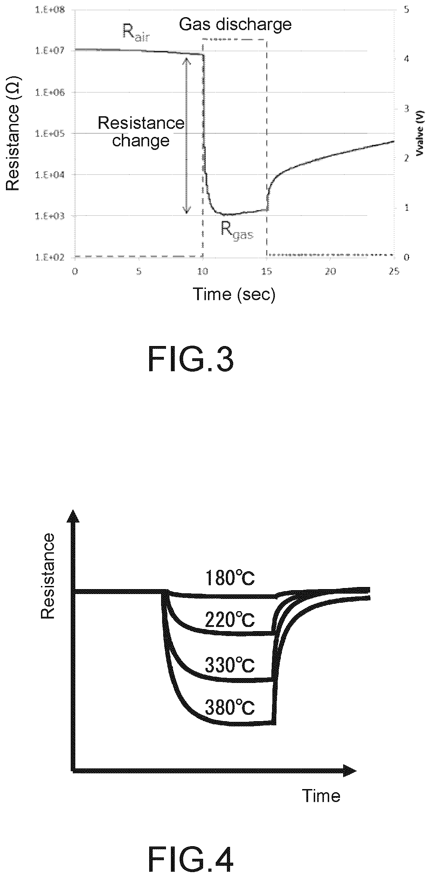

Fig. 3 shows an example of a time change of a resistance value Rs output from the semiconductor sensor 10. In this example, the semiconductor sensor 10 was driven in an air atmosphere in which a reducing gas is not present, and a reducing gas was discharged into the air atmosphere for an arbitrary time (five seconds in this example).

Note that the air atmosphere in which a reducing gas is not present means a clean air atmosphere. The clean air atmosphere is typically a dry air atmosphere in which the temperature and humidity are controlled, for example. Here, the phrase "temperature and humidity are controlled" represents a controlled state in which a specific substance is removed by passing through a filter such as activated carbon, silica gel, and a molecular sieve by a pump. The specific substance is, for example, water vapor or VOC (volatile organic compound) in the environment.

Further, the dry air atmosphere here represents an air atmosphere in which the humidity is controlled at an arbitrary value.

As shown in Fig. 3, a resistance value Rgas of the semiconductor sensor 10 in the air atmosphere in which a reducing gas is present is lower than a resistance value Rair of the semiconductor sensor 10 in the clean air atmosphere. When the discharge of the reducing gas is stopped, the output of the semiconductor sensor 10 returns to the resistance value (Rair) in the clean air atmosphere.

The greater the decrease in Rgas with respect to Rair, the greater the sensitivity to the reducing gas. In the following description, the ratio of Rair to Rgas (Rair/Rgas) is defined as the sensor sensitivity.

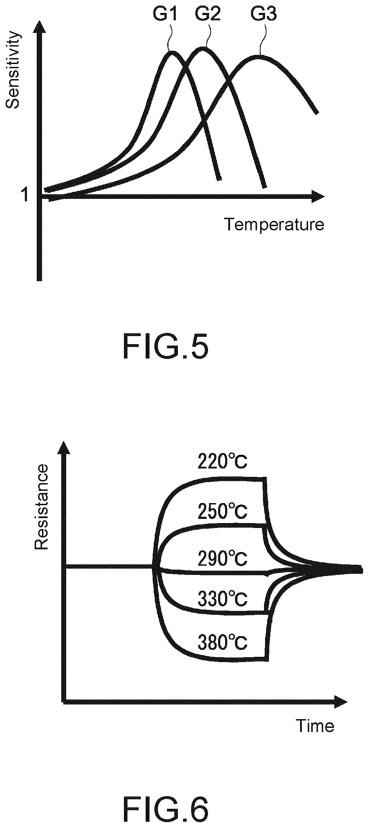

The resistance value and sensitivity of the semiconductor sensor 10 changes depending on the heating temperature of the heating layer 14. Fig. 4 is a schematic diagram showing a relationship between a heating temperature and a resistance value (hereinafter, referred to also as a resistance value profile). Fig. 5 is a schematic diagram showing a relationship between a heating temperature and sensitivity (hereinafter, referred to also as a sensitivity profile).

In the case of general reducing gases (e.g., hydrogen, acetone, ethanol, toluene, benzene, or cyclohexane), the semiconductor sensor 10 tends to have a larger amount of decrease in the resistance value and sensitivity of a value larger than one as the heating temperature rises as shown in Fig. 4 and Fig. 5. Further, as schematically shown in Fig. 5, it is possible to identify three different gas species G1, G2, and G3 on the basis of the difference in the sensitivity profile.

This type of change in the resistance value of the semiconductor sensor 10 due to the reducing gas is generally considered to be caused due to desorption/adsorption of oxygen accompanying oxidation-reduction of the metal oxide forming the adsorption layer 13.

For example, in the air atmosphere in which a reducing gas is not present, oxygen is adsorbed on the surface of the metal oxide particles to form a space charge layer in the vicinity of the surface of the particles. As a result, a potential barrier is formed at the grain boundary and hinders movement of electrons between the particles, thereby increasing the resistance value.

In this state, when a reducing gas is sprayed onto the semiconductor sensor 10, the oxygen adsorbed on the surface of the metal oxide particles is consumed, and thus, the space charge layer is thinned. As a result, the potential barrier is lowered, and thus, the resistance value is reduced.

Since the above-mentioned resistance value profile and sensitivity profile are unique to the gas species, it is theoretically possible to identify the gas species from the difference in these profiles. However, in the case where there is no sufficient difference in these profiles, it is difficult to identify the gas species with high accuracy.

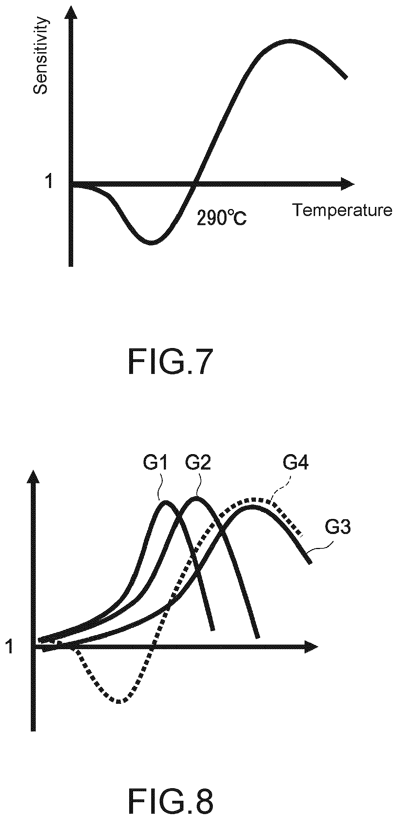

Meanwhile, the present inventors have found that a specific type of reducing gas has a specific resistance value profile and sensitivity profile as schematically shown I Fig. 6 and Fig. 7.

That is, regarding the resistance value, the resistance value is higher in a certain temperature range (220°C to 250°C in the example of Fig. 6) and the resistance value is lower in a temperature range (290°C to 380°C in the same) higher than the temperature range. When this is converted into sensor sensitivity, the sensitivity is less than one in the temperature range described above and the sensitivity is greater than one in the higher temperature range (approximately 290°C or more) as shown in Fig. 7.

Note that this is presumably because the gas species is adsorbed and attracts electrons from the semiconductor sensor in the low temperature range to increase the resistance and the gas species is decomposed in the high temperature range to decrease the resistance of the semiconductor sensor, although the details of the reason why such a specific profile appears are unknown.

As shown in Fig. 8, the gas species having a specific profile as described above is capable of easily identifying the specific gas species G4 by using the properties that the sensitivity shifts from decreasing to increasing.

[Experimental example]

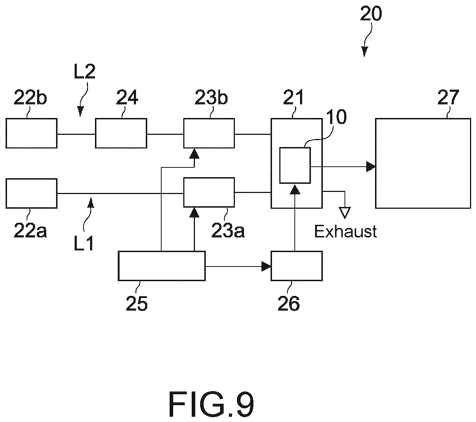

The present inventors measured a change in the resistance value of various reducing gases by the semiconductor sensor 10 using the gas detection system shown in Fig. 9. Fig. 9 is a schematic configuration diagram of a gas detection system 20.

The semiconductor sensor 10 is placed in a measurement chamber 21. A dry air introduction line L1 and a reducing gas introduction line L2 are connected to the measurement chamber 21. In order to maintain the pressure inside the measurement chamber 21 constant, the gas introduced into the measurement chamber 21 is discharged from the measurement chamber 21.

The dry air introduction line L1 is configured to be capable of driving a pump unit 22a to introduce dry air (temperature of 25°C and a relative humidity of 31%. The same applies hereinafter) into the measurement chamber 21 via a solenoid valve 23a.

The reducing gas introduction line L2 is configured to be capable of driving a pump unit 22b to introduce dry air into a gas chamber 24 filled with a reducing gas (saturated vapor) and introduce dry air containing a predetermined concentration of reducing gas into the measurement chamber 21 via a solenoid valve 23b.

The electrodes 12a and 12b of the semiconductor sensor 10 are each a rectangular Ti/Au film having a thickness of 200 nm and a size of 5 mm × 4.5 mm, and have been disposed on the substrate 11 that has a thickness of 525 um and is formed of alumina such that the long sides of the electrodes 12a and 12b face each other with the gap G of 5 um sandwiched therebetween. The drive voltage Vc (see Fig. 2) to be input between the electrodes 12a and 12b was set to 5 V. The adsorption layer 13 is an n-type metal oxide semiconductor ($WO_3$-Pd) containing a tungsten oxide as a metal oxide material and palladium as a catalyst metal material, and has a thickness of several um to 30 um.

As the pump units 22a and 22b, commercially available pumps having a flow rate of 400 ml/min were used. The solenoid valves 23a and 23b are open/close valves capable of switching between two positions of fully open and fully closed.

A control device 25 controls the solenoid valves 23a and 23b and a heater power source 26 that heats the heating layer 14 (see Fig. 1) of the semiconductor sensor 10.

The control device 25 opens one of the solenoid valves 23a and 23b and closes the other to select one of the dry air introduction line L1 and the reducing gas introduction line L2.

The control device 25 controls the heater power source 26 to control the heating temperature of the semiconductor sensor 10. The heating temperature of the semiconductor sensor 10 is arbitrarily adjusted within the range of 40°C to 500°C.

A measuring apparatus 27 acquires the resistance value Rs of the semiconductor sensor 10 at a plurality of temperatures within the heating temperature range on the basis of the output Vout (see Fig. 2) of the semiconductor sensor 10. The resistance value Rs corresponds to a resistance value in the air atmosphere in which a reducing gas is not present (Rair) when dry air is introduced from the dry air introduction line L1 into the measurement chamber 21 and a resistance value in the air atmosphere in which a reducing gas is present (Rgas) when a mixed gas of a reducing gas and dry air is introduced from the reducing gas introduction line L2 into the measurement chamber 21.

Using the gas detection system 20 configured as described above, the response properties of the semiconductor sensor 10 for a plurality of types of reducing gases were evaluated.

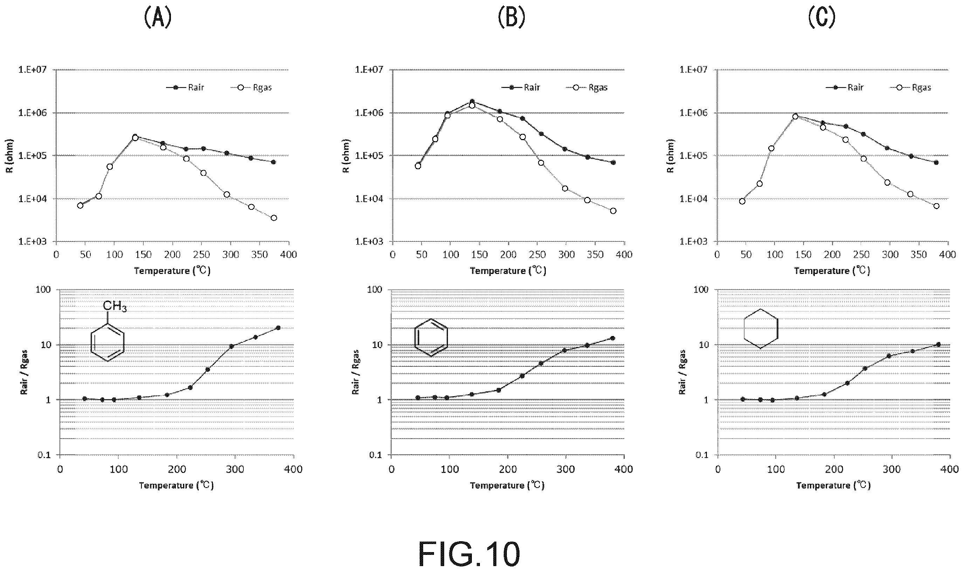

Fig. 10 shows the resistance value profile (upper) and the sensitivity profile (lower) of the semiconductor sensor 10 when (A) toluene, (B) benzene, and (C) cyclohexane are used as reducing gases.

As shown in the figure, these gas species show a change in the resistance value at a heating temperature of 100°C or more and show a tendency that the amount of decrease in the resistance value gradually increases as the heating temperature rises. Similarly, the sensor sensitivity tends to gradually increase as the heating temperature rises. The sensitivity profiles of these gas species match the aspects of the sensitivity profiles of the typical reducing gases shown in Fig. 5.

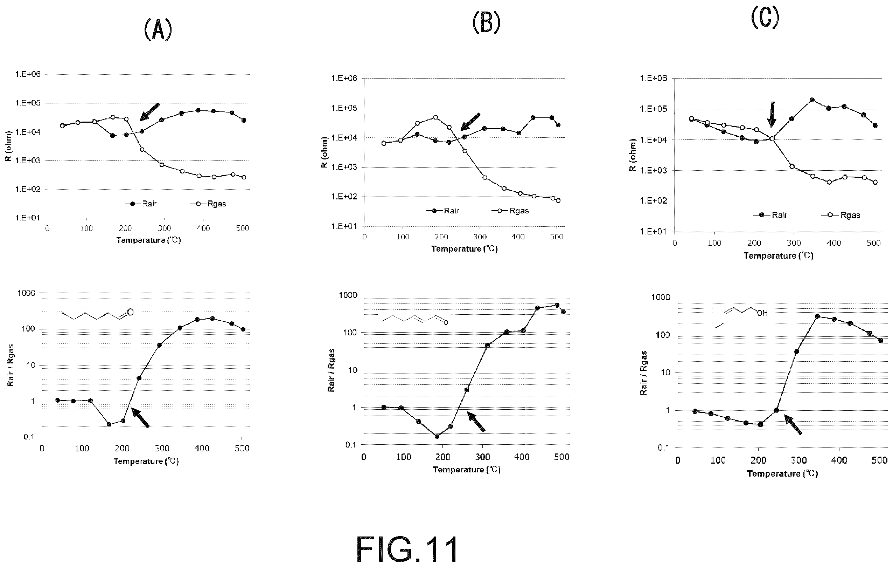

Meanwhile, Fig. 11 shows the resistance value profile (upper) and the sensitivity profile (lower) of the semiconductor sensor 10 when (A) n-hexanal, (B) (E)-2-hexenal, and (C) (Z)-3-hexenol are used as reducing gases.

As shown in Parts (A), (B), and (C) of Fig. 11, for any of n-hexanal, (E)-2-hexenal, and (Z)-3-hexenol, in the first temperature range, the measured value (Rgas) of the semiconductor sensor 10 during spraying of the specific gas species tends to be higher than the measured value (Rair) before the spraying. Meanwhile, in the second temperature range higher than the first temperature range, the magnitude relationship between Rgas and Rair is reversed (higher temperature than the arrow in the figure), and Rgas is lower than Rair.

Similarly, as shown in Parts (A), (B), and (C) of Fig. 11, the sensor sensitivity is reduced to be less than one in the first temperature range, but increases to a value larger than one in the second temperature range (higher temperature than the arrow in the figure).

Note that the first temperature range described above is approximately 120°C to approximately 220°C in the case of n-hexanal, approximately 100°C to approximately 250°C in the case of (E)-2-hexenal, and approximately 60°C to approximately 250°C in the case of (Z)-3-hexenol.

Examples of the gas species having a specific resistance value profile and sensitivity profile as shown in Parts (A), (B), and (C) of Fig. 11 include a hydrocarbon substance emitted from a plant, such as (Z)-3-hexenyl acetate, α-pinene, and methyl salicylate as described below, in addition to the above.

The change in the resistance value of various reducing gases by the semiconductor sensor 10 of (Z)-3-hexenylacetate and α-pinene as describe above is shown below.

Although the experimental conditions are substantially the same as those of the above-mentioned experimental example, the difference is that the metal oxide material of the adsorption layer 13 is a zinc oxide.

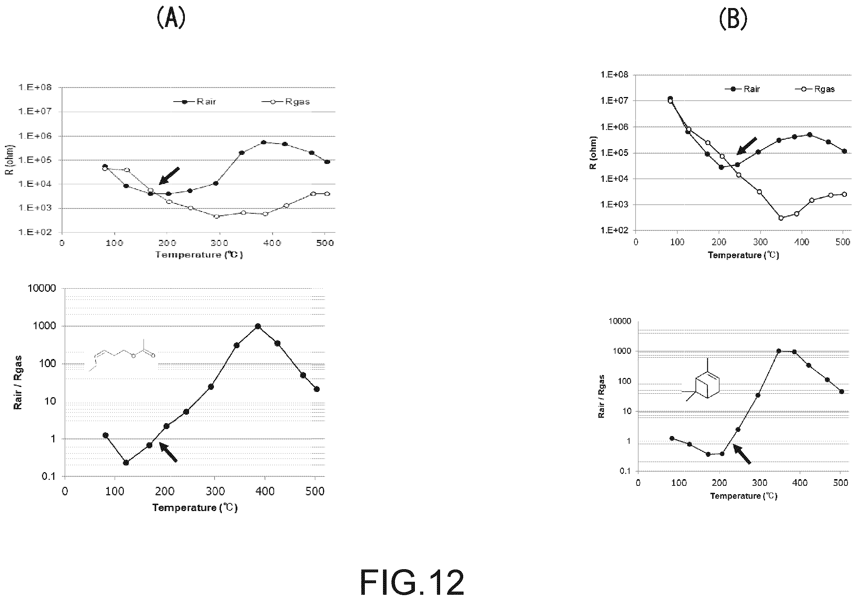

Then, Fig. 12 shows the resistance value profile (upper) and the sensitivity profile (lower) of the semiconductor sensor 10 when (A) (Z)-3-hexenylacetate and (B) α-pinene are used as reducing gases.

As shown in Parts (A) and (B) of Fig. 12, for any of (Z)-3-hexenylacetate and α-pinene, in the first temperature range, the measured value (Rgas) of the semiconductor sensor 10 during spraying of the specific gas species tends to be higher than the measured value (Rair) before the spraying. Meanwhile, in the second temperature range higher than the first temperature range, the magnitude relationship between Rgas and Rair is reversed (higher temperature than the arrow in the figure), and Rgas is lower than Rair.

Similarly, the sensor sensitivity is reduced to be less than one in the first temperature range, but increases to a value larger than one in the second temperature range (higher temperature than the arrow in the figure) as shown in Parts (A) and (B) of Fig. 12.

Note that the first temperature range described above is approximately 80°C to approximately 180°C in the case of (Z)-3-hexenylacetate and approximately 80°C to approximately 230°C in the case of α-pinene.

The change in the resistance value of the educing gas by the semiconductor sensor 10 of methyl salicylate as describe above is shown below.

Although the experimental conditions are substantially the same as those of the above-mentioned experimental example, the difference is that the adsorption layer 13 contains a tungsten oxide as a metal oxide material and rhenium as a catalyst metal material.

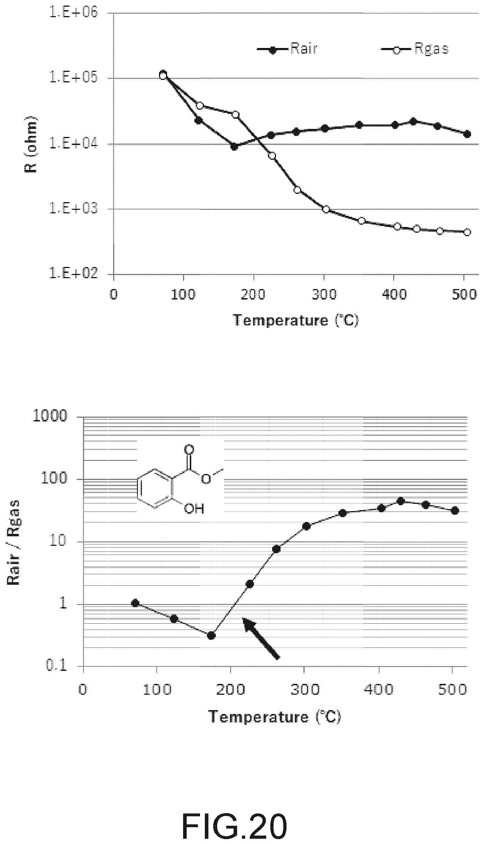

Then, Fig. 20 shows the resistance value profile (upper) and the sensitivity profile (lower) of the semiconductor sensor 10 when methyl salicylate is used as a reducing gas.

As shown in Fig. 20, also for methyl salicylate, in the first temperature range, the measured value (Rgas) of the semiconductor sensor 10 during spraying of the specific gas species tends to be higher than the measured value (Rair) before the spraying. Meanwhile, in the second temperature range higher than the first temperature range, the magnitude relationship between Rgas and Rair is reversed (higher temperature than the arrow in the figure), and Rgas is lower than Rair.

Similarly, the sensor sensitivity is reduced to be less than one in the first temperature range, but increases to a value larger than one in the second temperature range(higher temperature than the arrow in the figure) as shown in Fig. 20.

Note that the first temperature range described above is approximately 80°C to approximately 220°C.

[Gas detection method]

Application example of the semiconductor sensor 10 include a detection apparatus having a function of detecting plant damage by pests described below.

In the case where plants are cultivated throughout agriculture including plant factories, the above-mentioned gas detection method can also be applied to detect plant damages by pests.

It is known that plants release volatile molecules called green leaf volatiles (GLVs) when they are damaged by pests. The green leaf volatiles are the main component of green leaf scent and grassy smell, and approximately nine types, (Z)-3-hexenal, (Z)-3-hexenol, (Z)-3-hexenyl acetate, (E)-2-hexenal, (E)-2-hexenol, (E)-2-hexenyl acetate, n-hexanal, n-hexanol, and n-hexanyl acetate, are currently known. In addition, it is known that plants release volatile chemical substances that act to attract predators of herbivores (such as parasitic wasps), which are specifically produced when plants are damaged by pests or the like, such as herbivore-induced plant volatiles (HIPVs). The classification thereof includes many terpenes and terpenoids, it has the characteristic of releasing a unique blend of odors in accordance with the type of pest, and α-pinene, d-limonene, (Z)-β-ocimene, jasmonic acid, and the like are known.

In addition to the above, there is methyl salicylate as a volatile molecule that is likely to be released when plants are diseased (pathogen infection). Methyl salicylate is also known as a substance involved in plant-to-plant communication.

In the past, a method of preparing a molecular template polymer on which a specific volatile substance is adsorbed, causing a target volatile substance to be adsorbed on the molecular template polymer in advance, and detecting only the volatile substance by a MOx sensor has been known as a method of detecting damage caused by pests, but has the following problems.

·It is necessary to prepare a molecular template polymer for each volatile substance to be measured, and volatile substances having different molecular structures cannot be adsorbed by the same molecular template polymer.
-Since the molecular template polymer has a macromolecule, it has poor long-term stability under high temperature and high humidity conditions.

The detection method described above causes a volatile substance to be selectively adsorbed on a molecular template polymer and detects the volatile substance by a MOx sensor. However, in the present technology, a hydrocarbon substance emitted from plants, which is called "green leaf volatiles" or "herbivore-induced plant volatiles", is detected on the basis of the difference in temperature/sensitivity profile of the MOx sensor, and the hydrocarbon substance can be detected only by the MOx sensor without using a molecular template polymer.

Here, the hydrocarbon substance may be a C6 linear aliphatic compound, a terpene, or terpenoids, and the molecular weight thereof is typically 50 or more and 250 or less, and in the range of approximately 90 or more and 160 or less, specifically 98 or more and 152 or less in the case of the substance adopted in the experimental example described above.

In accordance with the above-mentioned gas detection method, by detecting the hydrocarbon substance described above, it is possible to detect plant damage by pests at an early stage and take countermeasures at an early stage.

In order to identify a gas species, the resistance value profile of the semiconductor sensor 10 may be referred to or the sensitivity profile may be referred to.

In the case of referring to a resistance value profile, the semiconductor sensor 10 including an adsorption layer formed of a metal oxide is heated, the resistance value (Rgas) of the semiconductor sensor 10 in the air atmosphere in which a reducing gas is present is measured, and it is determined, in the case where the resistance value (Rgas) is high in the first temperature range and low in the second temperature range higher than the first temperature range with reference to the resistance value (Rair) of the semiconductor sensor 10 in the air atmosphere in which a reducing gas is not present, that the reducing gas contains a hydrocarbon substance emitted from plants.

Meanwhile, in the case of referring to the sensitivity profile, the semiconductor sensor 10 including an adsorption layer formed of a metal oxide is heated, the resistance value (Rgas) of the semiconductor sensor 10 in the air atmosphere in which a reducing gas is present is measured, and it is determined, in the case where the sensitivity (Rair/Rgas) that is the ratio of the resistance value of the semiconductor sensor 10 in the air atmosphere in which a reducing gas is not present with respect to the measured resistance value (Rgas) is less than one in the first temperature range and is larger than one in the second temperature range higher than the first temperature range, that the reducing gas contains a hydrocarbon substance emitted from plants.

(Information processing apparatus)

In the above-mentioned gas detection system, the measuring apparatus 27 can be configured as a detection apparatus of a reducing gas containing a hydrocarbon substance emitted from plants.

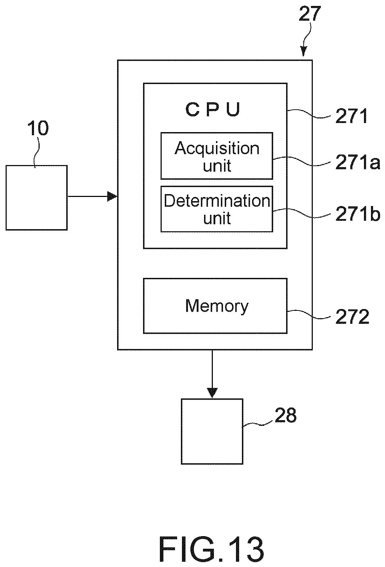

Fig. 13 is a schematic configuration diagram of the measuring apparatus 27. The measuring apparatus 27 includes a computer (information processing apparatus) including a CPU 271 and a memory 272.

The CPU 271 includes an acquisition unit 271a and a determination unit 271b. The acquisition unit 271a acquires the resistance value Rgas of the semiconductor sensor 10 in the air atmosphere in which a reducing gas is present.

The acquisition unit 271a may include a calculation unit that converts the output (Vout) of the semiconductor sensor 10 into the resistance value Rgas.

The determination unit 271a is configured to determine, on the basis of the resistance value Rgas of the semiconductor sensor 10 acquired by the acquisition unit 271a, whether or not the resistance value profile or sensitivity profile thereof is a predetermined profile.

The memory 272 is an information storage device such as a semiconductor memory and a hard disk. The memory 272 stores a program for causing the acquisition unit 271a and the determination unit 271b to operate as functional blocks of the CPU 271. Further, the memory 272 stores data relating to the resistance value profile and the sensitivity profile of the semiconductor sensor 10 in the air atmosphere in which a reducing gas is not present.

The output of the measuring apparatus 27 may be displayed on a display unit 28. The display unit 28 displays, for example, the resistance value profile and the sensitivity profile of the semiconductor sensor 10 as shown in Fig. 10 and Fig. 11.

(Processing procedure 1)

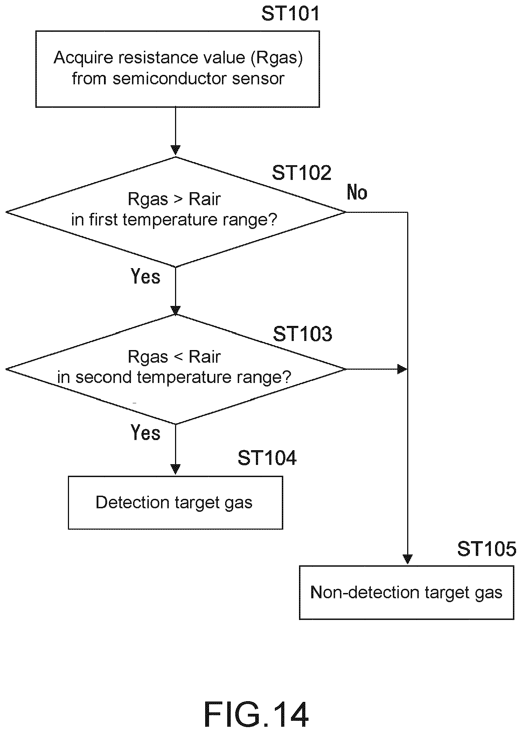

Fig. 14 is a flowchart showing an example of the processing procedure executed in the CPU 271.

First, the acquisition unit 271a acquires the resistance value (Rgas) of the semiconductor sensor 10 in the air atmosphere containing a reducing gas (Step 101) .

Subsequently, the determination unit 271b determines whether or not the measured resistance value (Rgas) is high in the first temperature range with reference to the resistance value (Rair) of the semiconductor sensor in the air atmosphere in which a reducing gas is not present (Step 102).

In the case where Rgas > Rair ("Yes" in Step 102), the determination unit 271b further determines whether or not the measured resistance value (Rgas) is low in the second temperature range higher than the first temperature range with reference to the resistance value (Rair) of the semiconductor sensor in the air atmosphere in which a reducing gas is not present (Step 103) .

Then, the determination unit 271b determines, in the case where both Steps 102 and 103 are "Yes", that the reducing gas is a detection target gas (gas containing a hydrocarbon substance emitted from plants) (Step 104) and determines, in the case where at least one of Steps 102 and 103 is "No", that the reducing gas is a non-detection target gas (gas that does not contain a hydrocarbon substance emitted from plants) (Step 105).

(Processing procedure 2)

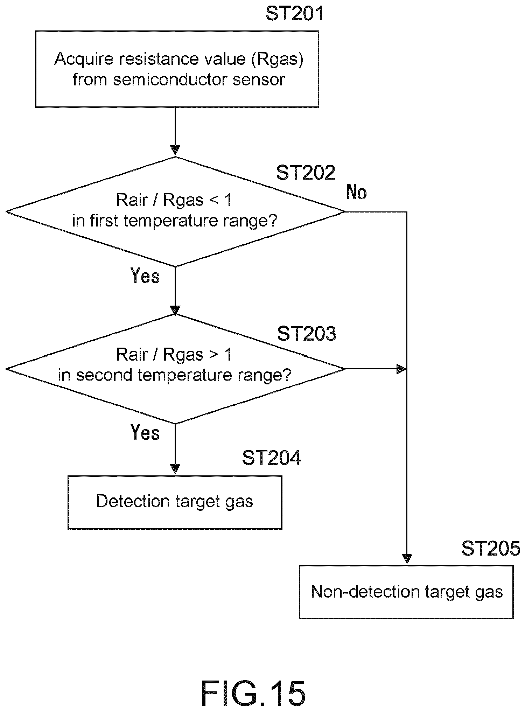

Fig. 15 is a flowchart showing another example of the processing procedure executed in the CPU 271.

First, the acquisition unit 271a acquires the resistance value (Rgas) of the semiconductor sensor 10 in the air atmosphere containing a reducing gas (Step 201) .

Subsequently, the determination unit 271b determines whether or not the sensitivity (Rair/Rgas) that is the ratio of the resistance value (Rair) of the semiconductor sensor in the air atmosphere in which a reducing gas is not present with respect to the measured resistance value (Rgas) is less than one in the first temperature range (Step 202).

In the case where Rair / Rgas < 1 ("Yes" in Step 202), the determination unit 271b further determines whether or not the sensitivity (Rair/Rgas) is larger than one in the second temperature range higher than the first temperature range (Step 203).

Then, the determination unit 271b determines, in the case where both Steps 202 and 203 are "Yes", that the reducing gas is a detection target gas (gas containing a hydrocarbon substance emitted from plants) (Step 204), and determines, in the case where at least one of Steps 202 and 203 is "No", that the reducing gas is a non-detection target gas (gas that does not contain a hydrocarbon substance emitted from plants) (Step 205).

[Method of diagnosing plant]

Other application examples of the semiconductor sensor 10 include a detection apparatus having a function of diagnosing plants as described below.

In an area in which one or more plants to be tested are grown, the above-mentioned gas detection method is applied to detect the presence or absence of a hydrocarbon substance emitted from plants, such as the above-mentioned green leaf volatiles and herbivore-induced plant volatiles, and diagnose the presence or absence of plant damage by pests.

In order to identify a gas species, the resistance value profile of the semiconductor sensor 10 may be referred to or the sensitivity profile may be referred to.

In the case of referring to a resistance value profile, the semiconductor sensor 10 including an adsorption layer formed of a metal oxide is heated, the resistance value (Rgas) of the semiconductor sensor 10 in the air atmosphere in which a reducing gas is present is measured, it is determined, in the case where the measured resistance value (Rgas) is high in the first temperature range and low in the second temperature range higher than the first temperature range with reference to the resistance value (Rair) of the semiconductor sensor 10 in the air atmosphere in which a reducing gas is not present, the reducing gas contains a hydrocarbon substance emitted from plants, and

the state of plants in the area in which one or more plants to be tested are grown is diagnosed on the basis of detection information of the hydrocarbon substance emitted from plants.

Meanwhile, in the case of referring to the sensitivity profile, the semiconductor sensor 10 including an adsorption layer formed of a metal oxide is heated, the resistance value (Rgas) of the semiconductor sensor 10 in the air atmosphere in which a reducing gas is present is measured, it is determined, in the case where the sensitivity (Rair/Rgas) that is the ratio of the resistance value of the semiconductor sensor 10 in the air atmosphere in which a reducing gas is not present with respect to the measured resistance value is less than one in the first temperature range and is larger than one in the second temperature range higher than the first temperature range, that the reducing gas contains a hydrocarbon substance emitted from plants, and

the state of plants in the area described above is diagnosed on the basis of detection information of the hydrocarbon substance emitted from plants.

(Plant diagnosing apparatus)

In the above-mentioned gas detection system, a plant diagnosing apparatus 27A can be configured as a detection apparatus of a reducing gas containing a hydrocarbon substance emitted from plants, such as the above-mentioned green leaf volatiles and herbivore-induced plant volatiles.

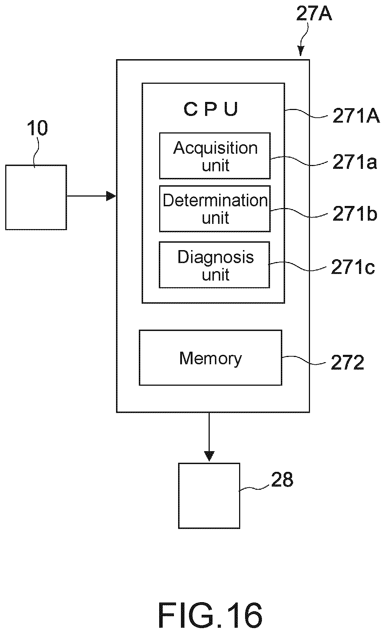

Fig. 16 is a schematic configuration diagram of the plant diagnosing apparatus 27A. The plant diagnosing apparatus 27A includes a computer (information processing apparatus) including the CPU 271 and the memory 272.

The CPU 271A includes the acquisition unit 271a, the determination unit 271b, and a diagnosis unit 271c. The acquisition unit 271a acquires the resistance value Rgas of the semiconductor sensor 10 in the air atmosphere in which a reducing gas is present.

The acquisition unit 271a may include a calculation unit that converts the output (Vout) of the semiconductor sensor 10 into the resistance value Rgas.

The determination unit 271b is configured to determine, on the basis of the resistance value Rgas of the semiconductor sensor 10 acquired by the acquisition unit 271a, whether or not the resistance value profile or the sensitivity profile is a predetermined profile.

The diagnosis unit 271c diagnoses the state of plants in the area in which one or more plants to be tested are grown by the determination result (detection information of a hydrocarbon substance emitted from plants) of the determination unit 271b.

The memory 272 is an information storage device such as a semiconductor memory and a hard disk. The memory 272 stores a program for causing the acquisition unit 271a, the determination unit 271b, and the diagnosis unit 271c to operate as functional blocks of the CPU 271. Further, the memory 272 stores the resistance value profile and the sensitivity profile of the semiconductor sensor 10 in the air atmosphere in which a reducing gas is not present.

The output of the plant diagnosing apparatus 27A may be displayed on the display unit 28. The display unit 28 displays, for example, the presence or absence of plant damage by pests by the diagnosis unit 271c.

(Diagnosis procedure 1)

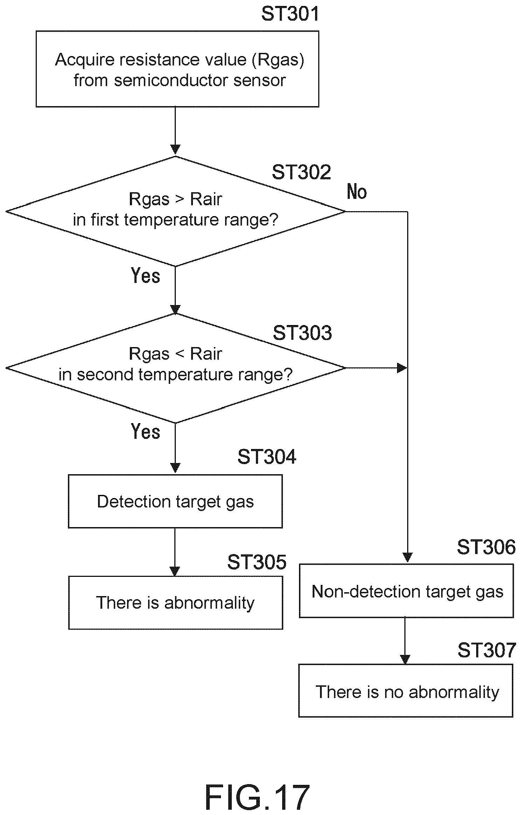

Fig. 17 is a flowchart showing an example of the diagnosis procedure executed in the CPU 271A.

Step 301 to Step304 and Step 306 shown in Fig. 17 are respectively similar to Step 101 to Step 104 and Step 105 of the above-mentioned processing procedure of the gas detection apparatus shown in Fig. 14, and the diagnosis unit 271c diagnoses, in the case where it is determined that a hydrocarbon substance emitted from plants is contained (Step304), that there is an abnormality (Step 305). Further, in the case where it is determined that a hydrocarbon substance emitted from plants is not contained (Step 306), the diagnosis unit 271c diagnoses that there is no abnormality (Step 307).

(Diagnosis procedure 2)

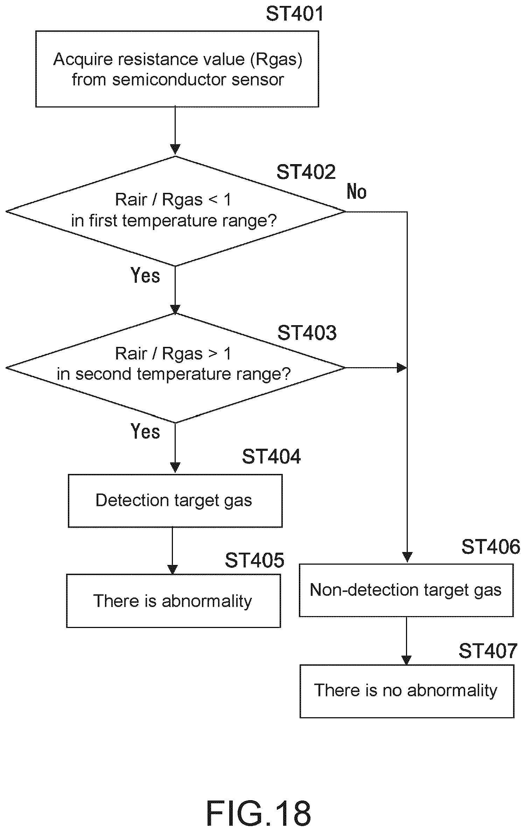

Fig. 18 is a flowchart showing another example of the diagnosis procedure executed in the CPU 271A.

Step 401 to Step 404 and Step 406 shown in Fig. 18 are similar to Step 201 to Step 204 and Step 205 of the above-mentioned processing procedure of the gas detection apparatus shown in Fig. 14, and the diagnosis unit 271c diagnoses, in the case where it is determined that a hydrocarbon substance emitted from plants is contained (Step 404), that there is abnormality (Step 405). Further, in the case where it is determined that a hydrocarbon substance emitted from plants is not contained (Step 406), the diagnosis unit 271c diagnoses that there is no abnormality (Step 407).

The first and second temperature ranges described above may be set in advance, but after acquiring all measured values at each temperature, whether or not there is an area corresponding to the first and second temperature ranges may be determined.

Further, the resistance value of the semiconductor sensor 10 at each heating temperature may be a resistance value measured at a predetermined sampling period while heating the semiconductor sensor 10 at a predetermined heating rate.

Alternatively, a plurality of semiconductor sensors 10 may be prepared and measured values when the plurality of semiconductor sensors is heated to different heating temperatures may be used.

<Modified example>

Although a hydrocarbon substance emitted from plants has been described above as an example in the example of the present technology, the present technology is not limited thereto. For example, a hydrocarbon substance emitted from animals may be detected by a MOx sensor according to the present technology. The hydrocarbon substance emitted from animals is, for example, nonanal, and can be detected by the MOx sensor to diagnose lung cancer.

<Application example>

Although a hydrocarbon substance emitted from plants is detected only by the MOx sensor in the example of the present technology, the present technology is not limited thereto.

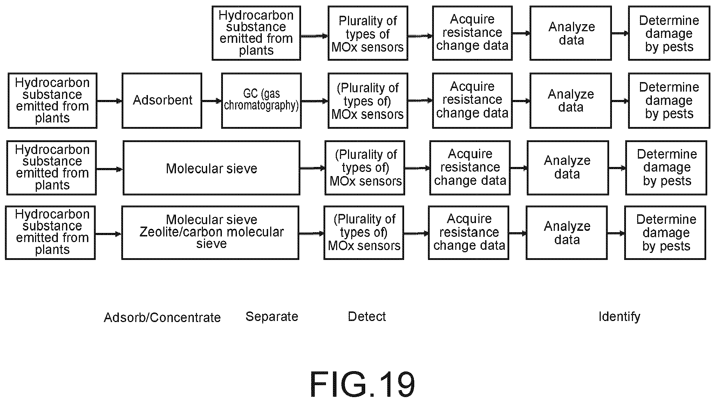

For example, Fig. 19 shows a process of determining the presence or absence of plant damage by pests. As shown in Fig. 19, a hydrocarbon substance emitted from plants may be adsorbed, concentrated, and separated by gas chromatography, molecular sieve (zeolite/carbon molecular sieve), or the above-mentioned molecular template polymer and then detected by the MOx sensor according to the present technology. As a result, it is possible to improve the detection accuracy of the MOx sensor according to the present technology.

It should be noted that the present technology may also take the following configurations.

(1) A gas detection method, including:

heating a semiconductor sensor that includes an adsorption layer formed of a metal oxide;
measuring a resistance value of the semiconductor sensor in an air atmosphere in which a reducing gas is present;
determining, where the measured resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

(2) The gas detection method according to (1) above, in which
the air atmosphere in which the reducing gas is not present is dry air.

(3) The gas detection method according to (1) or (2) above, in which

the semiconductor sensor includes a plurality of semiconductor sensors,
the step of heating the semiconductor sensor includes heating each of the plurality of semiconductor sensors to different heating temperatures, and
the step of measuring a resistance value of the semiconductor sensor includes measuring a resistance value of each of the plurality of semiconductor sensors.

(4) The gas detection method according to any one of (1) to (3) above, in which
the hydrocarbon substance is a substance containing a C6 linear aliphatic compound.

(5) The gas detection method according to any one of (1) to (4) above, in which
the hydrocarbon substance has a molecular weight of 90 or more and 160 or less.

(6) The gas detection method according to any one of (1) to (5) above, in which
the metal oxide is a sintered body containing a metal oxide material and a catalyst metal material.

(7) The gas detection method according to (6) above, in which

the metal oxide material includes a tungsten oxide, an indium oxide, a tin oxide, or a zinc oxide, and
the catalyst metal material includes iridium, an oxide thereof, palladium, an oxide thereof, rhenium, or an oxide thereof.

(8) A gas detection method, including:

heating a semiconductor sensor that includes an adsorption layer formed of a metal oxide;
measuring a resistance value of the semiconductor sensor in an air atmosphere in which a reducing gas is present;
determining, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from a plant or an animal.

(9) An information processing apparatus, including:

an acquisition unit that acquires a resistance value of a metal oxide semiconductor sensor in an air atmosphere in which a reducing gas is present; and
a determination unit that determines, where the acquired resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

(10) An information processing apparatus, including:

an acquisition unit that acquires a resistance value of a metal oxide semiconductor sensor in an air atmosphere in which a reducing gas is present; and
a determination unit that determines, where sensitivity that is a ratio of a resistance value of the semiconductor sensor in the air atmosphere in which the reducing gas is not present to the acquired resistance value is less than one in a first temperature range and greater than one in a second temperature range higher than the first temperature range, that the reducing gas contains a hydrocarbon substance emitted from a plant or an animal.

(11) A method of diagnosing a plant, including, in an area in which one or more plants to be tested are grown:

heating a semiconductor sensor that includes an adsorption layer formed of a metal oxide;
measuring a resistance value of the semiconductor sensor in an air atmosphere in which a reducing gas is present;
determining, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from a plant or an animal; and
diagnosing a state of the plants in the area on the basis of detection information of a hydrocarbon substance emitted from the plants.

(12) A method of diagnosing a plant, including, in an area in which one or more plants to be tested are grown:

heating a semiconductor sensor that includes an adsorption layer formed of a metal oxide;
measuring a resistance value of the semiconductor sensor in an air atmosphere in which a reducing gas is present;
determining, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from plants; and
diagnosing a state of the plants in the area on the basis of detection information of a hydrocarbon substance emitted from the plants.

(13) A plant diagnosing apparatus, including, in an area in which one or more plants to be tested are grown:

an acquisition unit that acquires a resistance value of a metal oxide semiconductor sensor in an air atmosphere in which a reducing gas is present;

a determination unit that determines, where sensitivity that is a ratio of a resistance value of the semiconductor sensor in the air atmosphere in which the reducing gas is not present to the acquired resistance value is less than one in a first temperature range and greater than one in a second temperature range higher than the first temperature range, the reducing gas contains a hydrocarbon substance emitted from plants; and

a diagnosis unit that diagnoses a state of the plants in the area on the basis of detection information of a hydrocarbon substance emitted from the plants obtained by the determination unit.

(14) A plant diagnosing apparatus, including, in an area in which one or more plants to be tested are grown;

an acquisition unit that acquires a resistance value of a metal oxide semiconductor sensor in an air atmosphere in which a reducing gas is present;

a determination unit that determines, where the acquired resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal; and

a diagnosis unit that diagnoses a state of the plants in the area on the basis of detection information of a hydrocarbon substance emitted from the plants obtained by the determination unit.

Reference Signs List

| | |
|---|---|
| 10 | semiconductor sensor |
| 11 | substrate |
| 12a, 12b | electrode |
| 13 | adsorption layer |
| 14 | heating layer |
| 20 | gas detection system |
| 27 | measuring apparatus |
| 271a | acquisition unit |
| 271b | determination unit |

**Claims**

1.  A gas detection method, comprising:

    heating a semiconductor sensor that includes an adsorption layer formed of a metal oxide;
    measuring a resistance value of the semiconductor sensor in an air atmosphere in which a reducing gas is present;
    determining, where the measured resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

2.  The gas detection method according to claim 1, wherein
    the air atmosphere in which the reducing gas is not present is dry air.

3.  The gas detection method according to claim 1, wherein

    the semiconductor sensor includes a plurality of semiconductor sensors,
    the step of heating the semiconductor sensor includes heating each of the plurality of semiconductor sensors to different heating temperatures, and
    the step of measuring a resistance value of the semiconductor sensor includes measuring a resistance value of each of the plurality of semiconductor sensors.

4.  The gas detection method according to claim 1, wherein
    the hydrocarbon substance is a substance containing a C6 linear aliphatic compound.

5. The gas detection method according to claim 1, wherein
the hydrocarbon substance has a molecular weight of 90 or more and 160 or less.

6. The gas detection method according to claim 1, wherein
the metal oxide is a sintered body containing a metal oxide material and a catalyst metal material.

7. The gas detection method according to claim 6, wherein

the metal oxide material includes a tungsten oxide, an indium oxide, a tin oxide, or a zinc oxide, and
the catalyst metal material includes iridium, an oxide thereof, palladium, an oxide thereof, rhenium, or an oxide thereof.

8. A gas detection method, comprising:

heating a semiconductor sensor that includes an adsorption layer formed of a metal oxide;
measuring a resistance value of the semiconductor sensor in an air atmosphere in which a reducing gas is present;
determining, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from a plant or an animal.

9. An information processing apparatus, comprising:

an acquisition unit that acquires a resistance value of a metal oxide semiconductor sensor in an air atmosphere in which a reducing gas is present; and
a determination unit that determines, where the acquired resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal.

10. An information processing apparatus, comprising:

an acquisition unit that acquires a resistance value of a metal oxide semiconductor sensor in an air atmosphere in which a reducing gas is present; and
a determination unit that determines, where sensitivity that is a ratio of a resistance value of the semiconductor sensor in the air atmosphere in which the reducing gas is not present to the acquired resistance value is less than one in a first temperature range and greater than one in a second temperature range higher than the first temperature range, that the reducing gas contains a hydrocarbon substance emitted from a plant or an animal.

11. A method of diagnosing a plant, comprising, in an area in which one or more plants to be tested are grown:

heating a semiconductor sensor that includes an adsorption layer formed of a metal oxide;
measuring a resistance value of the semiconductor sensor in an air atmosphere in which a reducing gas is present;
determining, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from a plant or an animal; and
diagnosing a state of the plants in the area on a basis of detection information of a hydrocarbon substance emitted from the plants.

12. A method of diagnosing a plant, comprising, in an area in which one or more plants to be tested are grown:

heating a semiconductor sensor that includes an adsorption layer formed of a metal oxide;
measuring a resistance value of the semiconductor sensor in an air atmosphere in which a reducing gas is present;
determining, where sensitivity that is a ratio of a resistance value the semiconductor sensor in the air atmosphere in which the reducing gas is not present to the measured resistance value is less than one in a first temperature range and greater than one in a second temperature range higher than the first temperature range, that the reducing gas contains a hydrocarbon substance emitted from plants; and

diagnosing a state of the plants in the area on a basis of detection information of a hydrocarbon substance emitted from the plants.

13. A plant diagnosing apparatus, comprising, in an area in which one or more plants to be tested are grown:

an acquisition unit that acquires a resistance value of a metal oxide semiconductor sensor in an air atmosphere in which a reducing gas is present;
a determination unit that determines, where sensitivity that is a ratio of a resistance value of the semiconductor sensor in the air atmosphere in which the reducing gas is not present to the acquired resistance value is less than one in a first temperature range and greater than one in a second temperature range higher than the first temperature range, the reducing gas contains a hydrocarbon substance emitted from plants; and
a diagnosis unit that diagnoses a state of the plants in the area on a basis of detection information of a hydrocarbon substance emitted from the plants obtained by the determination unit.

14. A plant diagnosing apparatus, comprising, in an area in which one or more plants to be tested are grown;

an acquisition unit that acquires a resistance value of a metal oxide semiconductor sensor in an air atmosphere in which a reducing gas is present;
a determination unit that determines, where the acquired resistance value is high in a first temperature range and low in a second temperature range higher than the first temperature range with reference to the resistance value of the semiconductor sensor in an air atmosphere in which the reducing gas is not present, that the reducing gas contains a hydrocarbon substance emitted from a plant or an animal; and
a diagnosis unit that diagnoses a state of the plants in the area on a basis of detection information of a hydrocarbon substance emitted from the plants obtained by the determination unit.

## FIG.1

## FIG.2

FIG.3

FIG.4

EP 4 317 956 A1

FIG.5

FIG.6

FIG.7

FIG.8

FIG.9

FIG.10

FIG.11

FIG.12

27

CPU — 271

Acquisition
unit — 271a

Determination
unit — 271b

Memory — 272

10

28

# FIG.13

ST101

Acquire resistance value (Rgas)
from semiconductor sensor

ST102

Rgas > Rair
in first temperature range?

No

Yes

ST103

Rgas < Rair
in second temperature range?

Yes

ST104

Detection target gas

ST105

Non-detection target gas

FIG.14

ST201

Acquire resistance value (Rgas)
from semiconductor sensor

ST202

Rair / Rgas < 1
in first temperature range?

No

Yes

ST203

Rair / Rgas > 1
in second temperature range?

Yes

ST204

Detection target gas

ST205

Non-detection target gas

FIG.15

27A

10

CPU — 271A

Acquisition
unit — 271a

Determination
unit — 271b

Diagnosis
unit — 271c

Memory — 272

28

FIG.16

ST301

Acquire resistance value (Rgas)
from semiconductor sensor

ST302

Rgas > Rair
in first temperature range?

No

Yes

ST303

Rgas < Rair
in second temperature range?

Yes

ST304

Detection target gas

ST305

There is abnormality

ST306

Non-detection target gas

ST307

There is no abnormality

FIG.17

ST401

Acquire resistance value (Rgas)
from semiconductor sensor

ST402

Rair / Rgas < 1
in first temperature range?

No

Yes

ST403

Rair / Rgas > 1
in second temperature range?

Yes

ST404

Detection target gas

ST405

There is abnormality

ST406

Non-detection target gas

ST407

There is no abnormality

FIG.18

30

| Hydrocarbon substance emitted from plants | | GC (gas chromatography) | Plurality of types of MOx sensors | Acquire resistance change data | Analyze data | Determine damage by pests |
|---|---|---|---|---|---|---|
| Hydrocarbon substance emitted from plants | Adsorbent | GC (gas chromatography) | (Plurality of types of) MOx sensors | Acquire resistance change data | Analyze data | Determine damage by pests |
| Hydrocarbon substance emitted from plants | Molecular sieve | | (Plurality of types of) MOx sensors | Acquire resistance change data | Analyze data | Determine damage by pests |
| Hydrocarbon substance emitted from plants | Molecular sieve Zeolite/carbon molecular sieve | | (Plurality of types of) MOx sensors | Acquire resistance change data | Analyze data | Determine damage by pests |

Adsorb/Concentrate     Separate     Detect                              Identify

FIG.19

FIG.20

## INTERNATIONAL SEARCH REPORT

| International application No. |
|---|
| **PCT/JP2022/008215** |

| **A. CLASSIFICATION OF SUBJECT MATTER** |
|---|
| *G01N 27/12*(2006.01)i; *A01G 7/00*(2006.01)i; *A01M 1/00*(2006.01)i<br>FI: G01N27/12 A; A01G7/00 603; A01M1/00 Q |
| According to International Patent Classification (IPC) or to both national classification and IPC |

| **B. FIELDS SEARCHED** |
|---|
| Minimum documentation searched (classification system followed by classification symbols)<br>G01N27/12; A01G7/00; A01M1/00 |
| Documentation searched other than minimum documentation to the extent that such documents are included in the fields searched<br>Published examined utility model applications of Japan 1922-1996<br>Published unexamined utility model applications of Japan 1971-2022<br>Registered utility model specifications of Japan 1996-2022<br>Published registered utility model applications of Japan 1994-2022 |
| Electronic data base consulted during the international search (name of data base and, where practicable, search terms used)<br>JSTPlus/JMEDPlus/JST7580 (JDreamIII) |

| **C. DOCUMENTS CONSIDERED TO BE RELEVANT** | | |
|---|---|---|
| Category* | Citation of document, with indication, where appropriate, of the relevant passages | Relevant to claim No. |
| A | PACZKOWSKI, Sebastian et al. Evaluation of Early Bark Beetle Infestation Localization by Drone-based Monoterpene Detection. Forests. 16 February 2021, vol. 12, 228, <URL: https://doi.org/10.3390/f12020228><br>entire text, all drawings | 1-14 |
| A | JP 11-304746 A (MATSUSHITA SEIKO CO LTD) 05 November 1999 (1999-11-05)<br>entire text, all drawings | 1-14 |
| A | JP 2005-134311 A (FUJI ELECTRIC FA COMPONENTS & SYSTEMS CO LTD) 26 May 2005 (2005-05-26)<br>entire text, all drawings | 1-14 |
| A | 松井健二 , みどりの香り研究の最前線, 香料, no. 244, December 2009, pp. 21-34<br>entire text, all drawings, (MATSUI, Kenji. Frontier of study on green leaf volatiles. The koryo.) | 1-14 |
| A | CN 106908488 A (AGRICULTURAL INFORMATION INSTITUTE OF CAAS) 30 June 2017 (2017-06-30)<br>entire text, all drawings | 1-14 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 May 2022** | **17 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| | International application No. |
|---|---|
| | **PCT/JP2022/008215** |

**C.    DOCUMENTS CONSIDERED TO BE RELEVANT**

| Category* | Citation of document, with indication, where appropriate, of the relevant passages | Relevant to claim No. |
|---|---|---|
| A | JP 2016-65868 A (MITSUI SHIPBUILDING ENG) 28 April 2016 (2016-04-28)<br>entire text, all drawings | 1-14 |
| A | KR 10-2021-0004484 A (INDUSTRY-ACADEMIC COOPERATION FOUNDATION, YONSEI UNIVERSITY) 13 January 2021 (2021-01-13)<br>entire text, all drawings | 1-14 |
| P, A | WO 2021/106615 A1 (SONY GROUP CORP) 03 June 2021 (2021-06-03)<br>entire text, all drawings | 1-14 |

Form PCT/ISA/210 (second sheet) (January 2015)

INTERNATIONAL SEARCH REPORT
Information on patent family members

International application No.

**PCT/JP2022/008215**

| Patent document cited in search report | | | Publication date (day/month/year) | Patent family member(s) | Publication date (day/month/year) |
|---|---|---|---|---|---|
| JP | 11-304746 | A | 05 November 1999 | (Family: none) | |
| JP | 2005-134311 | A | 26 May 2005 | (Family: none) | |
| CN | 106908488 | A | 30 June 2017 | (Family: none) | |
| JP | 2016-65868 | A | 28 April 2016 | (Family: none) | |
| KR | 10-2021-0004484 | A | 13 January 2021 | (Family: none) | |
| WO | 2021/106615 | A1 | 03 June 2021 | (Family: none) | |

Form PCT/ISA/210 (patent family annex) (January 2015)

34

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017223557 A
- JP 2001175969 A